# EUROPEAN PATENT APPLICATION

(11) **EP 0 529 868 A2**
(43) Date of publication of application: **03.03.1993**
(21) Application number: 92307346.4
(22) Date of filing: 11.08.1992
(51) Int. Cl.: C07C 69/14, C07C 67/08

(54) **Process for the production of methyl acetate**

(30) Priority: 24.08.1991 GB 9118304
(71) Applicant: BP Chemicals Limited, London EC2M 7BA (GB)
(72) Inventor: Perry, Adam Robert, Hull HU12 8DS (GB); Pummell, David William, Hull HU12 8DS (GB); Thomson, Alasdair Iain, Hull HU12 8DS (GB)
(74) Representative: Perkins, Nicholas David

(57) **Abstract**

In an integrated process for the production of methyl acetate for downstream utilisation in which methanol and acetic acid are reacted in an esterification reactor and the methyl acetate is recovered by distillation and the water recovered by azeotropic distillation, the process is operated in 'standby' mode by shutting off the methanol and acetic acid feeds to the esterification reactor and recycling the methyl acetate and water to the esterification reactor so that the process may be rapidly re-started.

## Description

This invention relates in general to a process for the production of methyl acetate by esterifying methanol with acetic acid and in particular to incorporation of such a process in an integrated process wherein the methyl acetate is used in downstream operations, for example in the production of acetic anhydride with or without the net co-production of acetic acid.

Acetic acid and acetic anhydride have been known as industrial chemicals for many years. Production of acetic anhydride constitutes the second largest end use for acetic acid. Acetic anhydride is widely employed in the production of cellulose acetate and other cellulose esters. Smaller quantities are used in the production of specialist esters, aspirin and pesticides. Acetic acid is used as a preservative and as an intermediate in the production of, for example, acetate esters.

The production of acetic acid by the liquid phase carbonylation of methanol is a well-known industrially operated process and is widely operated commercially. The carbonylation process, which is typically catalysed by rhodium and methyl iodide, is described in detail in, for example, GB 1233121. European patent application number EP-A-0087870 describes a modification in which acetic anhydride, with or without the net co-production of acetic acid, is obtained from methanol and carbon monoxide in a series of esterification, carbonylation and separation, steps. In more detail the latter process comprises:
(1) reacting methanol with recycle acetic acid in an esterification step to form an esterification product containing predominantly methyl acetate, water and optionally unreacted methanol,
(2) removing part of the water from the esterification product,
(3) reacting the esterification product still containing water with carbon monoxide in a carbonylation step in the presence as catalyst of free or combined metallic carbonylation catalyst and, as promoter, free or combined halogen to form a carbonylation product containing acetic acid and acetic anhydride,
(4) separating the carbonylation product by fractional distillation into a low boiling fraction containing carbonylation feed and volatile carbonylation promoter components, acetic acid and acetic anhydride fractions, and a higher boiling fraction containing carbonylation catalyst components,
(5) recycling the low boiling fraction containing carbonylation feed and carbonylation promoter components and the higher boiling fraction containing carbonylation catalyst components to the carbonylation step, and
(6) recycling at least part of the acetic acid fraction to the esterification step.

One process for the production of methyl acetate is described in our European patent application number EP-A-0060717 which describes a process for the production of methyl acetate which comprises reacting, in an esterification reaction vessel, methanol at elevated temperature with acetic acid in the presence of an esterification catalyst to form a product comprising methyl acetate and water, in a first distillation column, distilling from the product in the presence of an entrainer which is sparingly soluble in water and which forms a minimum boiling point azeotrope therewith, an overhead fraction comprising methyl acetate, entrainer and water, separating water from methyl acetate and entrainer and thereafter in a second distillation column separating methyl acetate as an overhead fraction from entrainer by distillation.

An alternative process for the production of methyl acetate is described in US patent US 4,889,950 which describes a process for preparing a carboxylic ester by reacting a carboxylic acid with a low-boiling alcohol, which comprises charging the reaction mixture to a 1st distillation column, withdrawing the reaction components carboxylic ester, water and unconverted alcohol, but not unreacted carboxylic acid, as overhead product and charging this overhead product to a 2nd distillation column, from which (a) the overhead product comprises the alcohol or an azeoester, this overhead product being recycled into the esterification reaction, (b) a liquid side product comprising carboxylic ester and water is withdrawn from the stripping section and separated in a phase separating vessel into water and carboxylic ester and the carboxylic ester is returned into the distillation column below the side takeoff point, and (c) the bottom product removed is the carboxylic ester. According to the example described in US 4,889,950, a sulphuric acid catalyst was used.

A preferred method of production of methyl acetate suitable for operation as step (1) of the process of EP-A-0087870 is described in our European Patent EP-B-0060719, which describes a process for the production of methyl acetate comprising reacting in an esterification reaction vessel methanol at elevated temperature with acetic acid in the presence of an esterification catalyst and an entrainer which is sparingly soluble in water and which forms a minimum boiling point azeotrope therewith to form a product comprising entrainer, methyl acetate and water, and in a distillation column, recovering from the product an overhead fraction comprising methyl acetate characterised in that from an intermediate point in the column there is removed a liquid sidestream fraction comprising water and entrainer, the entrainer being recycled to the reaction vessel or distillation column. A particularly preferred embodiment of this invention is said to comprise continuously feeding methanol and acetic acid to a reaction kettle surmounted by a distillation column, which kettle contains entrainer, acetic acid and esterification catalyst at elevated temperature, thereby producing a product comprising methyl acetate, water, unreacted methanol, unreacted acetic acid, catalyst and entrainer, removing from the column an overhead fraction comprising principally methyl acetate together with some methanol and water, and condensing the overhead fraction, characterised in that the process comprises returning a portion of the condensed overhead fraction to the column as primary reflux leaving in the reaction kettle a residue fraction comprising catalyst, acetic acid, entrainer and some water, removing from an intermediate point in the column a liquid side-stream fraction comprising water, methyl acetate and entrainer together with some methanol, separating the sidestream fraction by decantation into an upper organic phase comprising principally entrainer and some methyl acetate and a lower aqueous phase comprising principally water and some methyl acetate and methanol and returning the separated organic phase to the column at a point in the column lower than the sidestream fraction removal point.

In an integrated process wherein the methyl acetate recovered from the esterification process is used in downstream operations, for example the production of acetic anhydride by the carbonylation process of EP-A-0087870 it is sometimes necessary to shut down the downstream operation. In such circumstances it is desirable that the esterification process be put on 'standby' or 'hold', the alternatives being either to shut down the esterification process every time the downstream operations are interrupted or to provide intermediate product storage, both of which options are economically disadvantageous.

We have now developed a method of operating the esterification process in a 'standby' mode whereby in a very short space of time it can be re-started, thereby facilitating rapid start up of downstream operations.

Thus according to the present invention there is provided a process for the production of methyl acetate which process is a step in an integrated process utilising methyl acetate downstream and which process comprises continuously feeding methanol and acetic acid to an esterification reactor to form, at elevated temperature and in the presence of an esterification catalyst, a product comprising methyl acetate and water; recovering methyl acetate from the esterification product by distillation and passing the recovered methyl acetate, with or without further purification, to downstream operation; and recovering water from the esterification product by azeotropic distillation and removing from the process the recovered water, with or without further purification; characterised in that the process is operated in 'standby' mode by the steps comprising:
(a) shutting off the methanol and acetic acid feeds to the esterification reactor; (b) recycling the recovered methyl acetate to the esterification reactor instead of passing it to downstream operation; and (c) recycling the recovered water to the esterification reactor instead of removing it from the process.

The process of the present invention solves the technical problem defined above by recycling the esterification products to the esterification reactor where, at elevated temperature and in the presence of the esterification catalyst, a reaction composition is maintained which allows the product recovery distillation steps to continue to operate whilst the process is operated in 'standby' mode. This enables the process to be re-started in a very short space of time, thereby facilitating rapid start up of downstream operations. In placing the process in 'standby' mode it may be necessary to adjust reflux of the distillations to prevent dumping.

The integrated process is preferably a process for the production of acetic anhydride with or without the net co-production of acetic acid by carbonylation of methyl acetate. Details of normal operation of such a process are described in EP-A-0087870, which is incorporated herein by reference.

The esterification process of the present invention may be any suitable esterification process which in normal operation to produce methyl acetate for downstream operations as opposed to 'standby' mode, involves the continuous reaction of methanol and acetic acid at elevated temperature in the presence of a esterification catalyst in an esterification reactor to produce methyl acetate and water, the methyl acetate being recovered by distillation for downstream use and the water being recovered by azeotropic distillation. Suitable processes are described in EP-A-0060717; US 4;889,950 and EP-B-0060719.

According to a first embodiment of the present invention the esterification process may be operated to produce methyl acetate for downstream utilization in an integrated-process scheme,-as opposed to 'standby' operation as described in the aforesaid European patent application EP-A-0060717, the contents of which are hereby incorporated by reference. Thus, according to this embodiment, methanol is reacted in an esterification reactor at elevated temperature with acetic acid in the presence of an esterification catalyst to form a product comprising methyl acetate and water; the esterification product is distilled in a first distillation column in the presence of an entrainer which is sparingly soluble in water and which forms a minimum boiling point azeotropic therewith to form an overhead fraction comprising methyl acetate, entrainer and water; water is separated from the methyl acetate and entrainer and thereafter methyl acetate is separated as an overhead fraction from the entrainer by distillation in a second distillation column. The entrainer may be returned to the esterification reaction vessel or the first distillation column. In this embodiment the esterification catalyst may suitably be a mineral acid such as sulphuric acid or an organic acid such as toluene-para-sulphonic acid. The entrainer for azeotropic distillation of the water may be any hydrocarbon, ether, ester or ketone which is sparingly soluble in water and which forms a minimum boiling point azeotrope with water. Further details of operation of this esterification process are to be found in EP-A-0060717.

The process of this embodiment is operated in 'standby' mode according to the present invention by the steps comprising (i) shutting off the methanol and acetic acid feeds to the esterification reactor; and (ii) recycling to the esterification reactor the methyl acetate and water distilled from the esterification reactor product. The methyl acetate may be recycled to the esterification reactor together with entrainer, in which case the second distillation column is by-passed and either shutdown or placed on 'hold' according to conventional procedures.
Alternatively, the second distillation column may be operated to separate the methyl acetate from the entrainer, the methyl acetate being recycled to the esterification reactor and the entrainer being recycled to the first distillation column and/or esterification reactor.

According to a second embodiment of the present invention the esterification process may be operated to produce methyl acetate for downstream utilisation in an integrated process scheme, as opposed to 'standby' operation as described in the aforesaid US patent US 4,889,950, the contents of which are hereby incorporated by reference. Thus, according to this embodiment a carboxylic ester is prepared by reacting a carboxylic acid with a low-boiling alcohol in the presence of an esterfication catalyst such as sulphuric acid, which comprises charging the reaction mixture to a 1st distillation column, withdrawing the reaction components carboxylic ester, water and unconverted alcohol, but not unreacted carboxylic acid, as overhead product and charging this overhead product to a 2nd distillation column, from which (a) the overhead product comprises the alcohol or an azeoester, this overhead product being recycled into the esterification reaction, (b) a liquid side product comprising carboxylic ester and water is withdrawn from the stripping section and separated in a phase separating vessel into water and carboxylic ester and the carboxylic ester is returned into the distillation column below the side takeoff point, and (c) the bottom product removed is the carboxylic ester. The process is placed on 'standby' mode by shutting off the alcohol and acid feeds to the esterification reactor and recycling the ester and water to the esterification reactor.

According to a third, preferred embodiment of the present invention the esterification process may be operated to produce methyl acetate for downstream utilisation in a integrated process scheme, as opposed to 'standby' operation as described in the aforesaid European Patent No. EP-B-0060719, the contents of which are hereby incorporated by reference. Thus, according to this embodiment of the present invention methanol is reacted at elevated temperature with acetic acid in an esterification reaction vessel in the presence of a esterification catalyst and an entrainer which is sparingly soluble in water and which forms a minimum boiling point azeotrope therewith to form an esterification product comprising entrainer, methyl acetate and water, an overhead fraction comprising methyl acetate is recovered from the esterification product in a distillation column and at an intermediate point in the column there is removed a liquid sidestream fraction comprising water and entrainer, the entrainer being recycled to the reaction vessel and/or distillation column. The process of this embodiment is operated in 'standby' mode according to the present invention by shutting off the methanol and acetic acid feeds to the esterification reaction vessel and recycling the methyl acetate and water to the esterification reaction vessel.

According to a preferred embodiment of the third embodiment of the present invention there is provided a process for the production of methyl acetate, the process being a step in an integrated process utilising methyl acetate downstream, and the process comprising continuously feeding methanol and acetic acid to an esterification reactor, said reactor containing (i) an entrainer which is sparingly soluble in water and which forms a minimum boiling point azeotrope therewith, (ii) acetic acid and (iii) an esterification catalyst, to produce a product comprising principally methyl acetate, unreacted methanol, entrainer and water, distilling from the product in a distillation column an overhead fraction comprising principally methyl acetate, condensing the overhead fraction, returning a portion of the overhead fraction to the column as primary reflux and passing remaining methyl acetate, with or without further purification, to downstream operation, removing from an intermediate point in the column a liquid sidestream fraction comprising water, methyl acetate and entrainer together with some methanol, cooling the sidestream fraction, separating the liquid sidestream fraction by decantation into an upper organic phase comprising principally entrainer and some methyl acetate and a lower aqueous phase comprising principally water and some methyl acetate and methanol, returning the separated organic phase to the column at a point lower in the column than the sidestream fraction removal point and passing the separated aqueous phase to a stripping column wherein a base aqueous fraction is separated from an overhead fraction, the base aqueous fraction being removed from the process and the overhead fraction being recycled to the esterification reactor and/or distillation column
characterised in that
the process is operated in 'standby' mode by the steps comprising:-
(i) shutting off the methanol and acetic acid feeds to the esterification reactor,
(ii) recycling substantially all the remaining methyl acetate separated overhead by distillation to the esterification reactor instead of passing it to downstream operation,
(iii)recycling the water in the lower aqueous phase from the decantation stage to the esterification reactor, and
(iv) adjusting the distillation column reflux to prevent dumping occurring on the distillation column.

This preferred embodiment is operated in 'standby' mode according to the present invention by the steps comprising:-
(i) shutting off the methanol and acetic acid feeds to the esterification reactor,
(ii) recycling substantially all the methyl acetate separated by distillation to the esterification reactor instead of passing it to downstream operation,
(iii)recycling the water in the aqueous phase from the decantation stage to the esterification reactor, and
(iv) adjusting the distillation column reflux to prevent dumping occurring on the distillation column.

In the preferred embodiment the water in the lower aqueous phase may be recycled to the esterification reactor by by-passing the stripping column and returning the phase directly to the esterification reactor, the stripping column either being shut down or held on stand-by at or near reflux without feeds or take-offs in conventional manner. Alternatively, in a preferred embodiment the lower aqueous phase may be passed through the stripping column which continues to operate; the base water fraction being returned to the esterification reactor and the overhead fraction being returned to the esterification reactor and/or distillation column. This reduces the effect of starting up and shutting down the stripping column on the esterification reactor operation, particularly when the stripper is not provided with a source of reflux other than the feed so that it cannot operate on 'stand-by' at or near reflux.

When a demand for methyl acetate downstream arises the process is readily converted once again from the 'standby' mode to the operational mode by (a) restarting the methanol and acetic acid feeds to the esterification reactor, (b) passing all the methyl acetate separated overhead by distillation to downstream operation, (c) ceasing recycle of the water in the lower aqueous phase from the decantation stage to the esterification reactor and optionally restarting the stripping column if it has been placed on 'stand-by' or shut down, and (d) adjusting the distillation column reflux to its normal value.

In the third embodiment of the present invention the entrainer may suitably be added to the esterification reactor or to a suitable point in the distillation column. The entrainer may be any hydrocarbon, ether, ester or ketone which is sparingly soluble in water and which forms a minimum boiling point azeotrope with water. Examples of suitable entrainers are toluene, diisobutyl ether, normal-and iso-butyl acetate and methyl isobutyl ketone. Preferably the entrainer is an acetic acid ester such as n-butyl acetate. An advantage of using an acetic acid ester is that it can be formed 'in situ', for example n-butyl acetate can be formed by incorporating n-butanol in the reaction mixture. Methyl acetate (b.pt. 57°C) and butyl acetate (b.pt.126°C) can be readily separated by distillation. The amount of entrainer present may suitably be greater than 1%, preferably from 20 to 60% by weight based on the total weight of the reaction mixture. Since, substantially all the entrainer remains within the column, only small amounts being lost in the overhead fraction and the sidestream fraction aqueous phase, it is preferred to feed entrainer in small amounts to compensate for these losses to the esterification reactor.

In the third embodiment of the present invention the esterification catalyst may suitably be a mineral acid, such as sulphuric acid, or an organic acid, such as para-toluene-sulphonic acid or methane-sulphonic acid, of which methane-sulphonic acid is preferred. The esterification catalyst may be present in an amount from 0.1 to 10%, preferably from 2 to 6% by weight of the reaction mixture.

Suitably the feed to the esterification reactor in the third embodiment comprises equimolar amounts of methanol and acetic acid. It is immaterial whether the methanol and/or acetic acid originally fed to the esterification reactor contain water. The esterification reactor may be separate from the distillation column or integral therewith. Suitably the esterification reactor comprises the reboiler kettle of the distillation column. The distillation column may suitably contain not less than 8 theoretical plates and preferably from 15 to 50 theoretical plates. Whenever the esterification reactor is separate from the distillation column it is preferred to recycle the residue from the base of the column to the esterification reactor. The distillation column may suitably incorporate means for facilitating the removal of a liquid sidestream fraction, which means may take the form of a deep weir or chimney tray located immediately below the point in the column from which the sidestream fraction is removed. Using such means it may be possible to maximise the concentration of water in the sidestream fraction by facilitating phase separation within the column, thereby saving energy.

Further details of the process when operating to produce methyl acetate for downstream use in an integrated process, as opposed to 'standby' operation may be found in the aforesaid European Patent No. EP-B-0060719, the contents of which are incorporated by reference herein. Typically, the esterification reaction is conducted at a temperature in the range from 90 to 120°C, preferably from 95 to 115°C. Suitably the primary reflux ratio may be in the range 1:2 to 10:1, preferably from 1:1 to 5:1. Operating at atmospheric pressure the sidestream fraction is preferably removed at a point in the distillation column at which the column temperature is between 75 and 90°C. In terms of theorectical plates this point should suitably be not less than 5 and preferably not less than 10 theoretical plates from the base of the column.

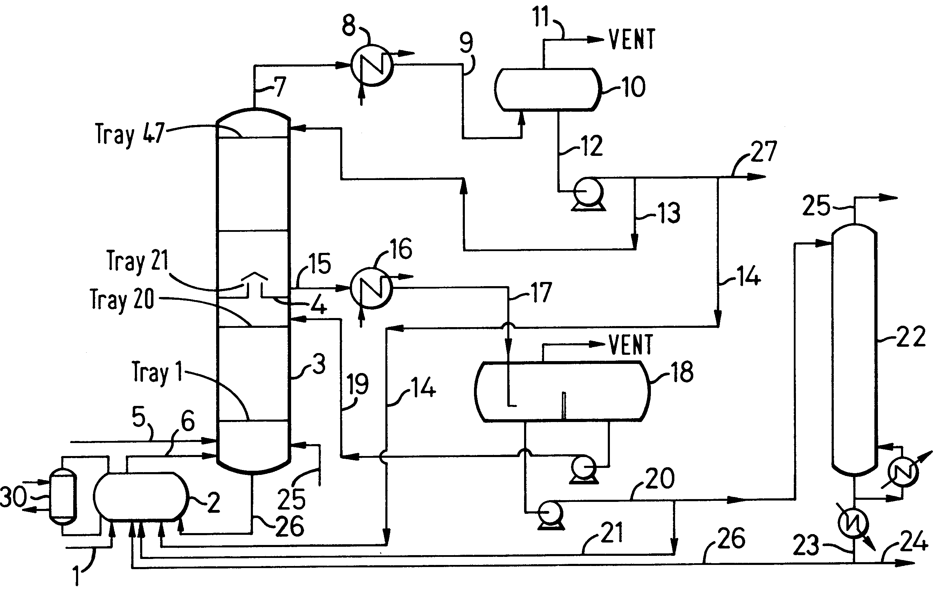

A preferred embodiment of the process of the present invention is further illustrated with reference to the accompanying drawing which is a simplified flow diagram.

In normal operation methanol is fed through line 1 to an esterification reactor 2 containing methane-sulphonic acid as esterification catalyst, n-butyl acetate as entrainer and acetic acid. The reactor 2 is connected to the distillation column 3 by lines 6 and 26. The reactor 2 and distillation column 3 are heated by reboiler 30. The reactor functions as a kettle for the distillation column 3 which contains 47 trays, including as tray 21 a chimney tray 4 (the column tray numbers are counted from the bottom to the top). Acetic acid is fed to the distillation column 3 through line 5 and thence to the reactor 2 through line 26 with liquid from the distillation column. A fraction comprising unreacted methanol, water, methyl acetate and n-butyl acetate is taken overhead from the reactor 2 through line 6 to the distillation column. Overhead from the distillation column 3 is taken through line 7, a vapour fraction comprising principally methyl acetate and small amounts of methanol, n-butyl acetate, acetic acid and water. This fraction is condensed in the condensor 8 and passed through line 9 to a reflux drum 10, which is vented through line 11. The methyl acetate removed from the drum through line 12 is divided into portions. A first portion is recycled through line 13 as primary reflux to the top of the distillation column 3. A second portion is passed along line 27 to downstream operation for example a carbonylation process as described in EP-A-0087870. A liquid sidestream comprising n-butyl acetate, methyl acetate, methanol, acetic acid (trace) and water is removed from the deep weir formed by the chimney tray 4 through line 15, passed through the cooler 16 and fed to a decantation vessel 18 through line 17. In the decantation vessel 18 separation into an oil phase and an aqueous phase occurs. The oil phase comprising principally n-butyl acetate, is taken from the decantation vessel 18 and is recycled through line 19 to tray 20 of the distillation column 3. The aqueous phase principally containing water and methyl acetate is taken from the decantation vessel 18 and passed through line 20 to a stripper column 22 from which a base aqueous fraction is recovered along line 23 and during normal operation is passed through line 24 to the waste. An overhead fraction is recovered from the stripper column and passed along line 25 to the base of the distillation column.

The process is operated in 'stand-by' mode by the steps:
(i) shutting off the methanol and acetic acid feeds along lines 1 and 5 respectively to the esterification reactor,
(ii) recycling substantially all the remaining methyl acetate separated overhead by distillation to the esterification reactor along line 14 instead of passing it to downstream operation,
(iii)either shutting down the stripper column and recycling the lower aqueous phase from the decanter 18 to the esterification reactor 2 along line 21 instead of to the stripper column, the stripper in this case being unable to operate in a stand-by mode because of the absence of reflux other than feed, or continuing to operate the stripper column 22, recycling the base aqueous fraction along line 26 to the esterification reactor instead of to waste along line 24, and continuing to recycle the overhead fraction along line 25 to the distillation column, and
(iv) adjusting the distillation column 3 reflux to prevent dumping occuring on the distillation column.

The process is returned to normal operation by:
(a) restarting the methanol and acetic acid feeds along lines 1 and 5 respectively to the esterification reactor,
(b) passing all the methyl acetate separated overhead by distillation to downstream operation along line 27, and either ceasing direct recycle of the lower aqueous phase from the decanter 18 to the esterification reactor and restarting the stripper column, or continuing to operate the stripper column and ceasing recycle of the base aqueous fraction from the stripper column to the esterification reactor, and
(d) adjusting the distillation column reflux to its normal operating value.

## Claims

1. A process for the production of methyl acetate which process is a step in an integrated process utilising methyl acetate downstream and which process comprises the steps of: continuously feeding methanol and acetic acid to an esterification reactor to form, at elevated temperature and in the presence of an esterification catalyst, a product comprising methyl acetate and water; recovering methyl acetate from the esterification product by distillation and passing the recovered methyl acetate, with or without further purification, to downstream operation; and recovering water from the esterification product by azeotrope distillation and removing from the process the recovered water, with or without further purification; characterised in that the process is operated in 'standby' mode by the steps comprising:
(a) shutting off the methanol and acetic acid feeds to the esterification reactor;
(b) recycling the recovered methyl acetate to the esterification reactor instead of passing it to downstream operation; and
(c) recycling the recovered water to the esterification reactor instead of removing if from the process.

2. A process as claimed in claim 1 in which when methyl acetate is produced for downstream utilisation an overhead fraction comprising methyl acetate, entrainer and water is distilled from the esterification product in a first distillation column in the presence of an entrainer which is sparingly soluble in water and which forms a minimum boiling point azeotrope therewith; water is separated from the methyl acetate and entrainer and methyl acetate is separated as an overhead fraction from the entrainer by distillation in a second distillation column.

3. A process as claimed in claim 1 in which when methyl acetate is produced for downstream utilisation, the esterification product is charged to a first distillation column from which is withdrawn as overhead product carboxylic ester, water and unconverted alcohol but not unreacted acid and in which this overhead product is charged to a second distillation zone from which an overhead product comprising alcohol or an azeoester is recycled to the esterification reaction; a liquid side product comprising carboxylic ester and water is withdrawn and separated in a phase separating vessel into water and carboxylic ester, the ester being returned to the distillation column below the side take off point and the bottom product removed is carboxylic ester.

4. A process as claimed in claim 1 in which when the process is operated to produce methyl acetate for downstream utilisation, methanol is reacted at elevated temperature with acetic acid in an esterification reaction vessel in the presence of an esterification catalyst and an entrainer which is sparingly soluble in water and which forms a minimum boiling point azeotrope therewith, to form an esterification product comprising entrainer, methyl acetate and water and in a distillation column there is recovered from the esterification product an overhead fraction comprising methyl acetate and from an intermediate point in the column there is removed a liquid sidestream comprising water and entrainer, the entrainer being recycled to the reaction vessel and/or distillation column.

5. A process for the production of methyl acetate the process being a step in an integrated process utilising methyl acetate downstream and the process comprising continuously feeding methanol and acetic acid to an esterification reactor, said reactor containing (i) an entrainer which is sparingly soluble in water and which forms a minimum boiling point azeotrope therewith, (ii) acetic acid and (iii) an esterification catalyst, to produce a product comprising principally methyl acetate, unreacted methanol, entrainer and water, distilling from the product in a distillation column an overhead fraction comprising principally methyl acetate, condensing the overhead fraction, returning a portion of the overhead fraction to the column as primary reflux and passing remaining methyl acetate, with or without further purification, to downstream operation, removing from an intermediate point in the column a liquid sidestream fraction comprising water, methyl acetate and entrainer together with some methanol, cooling the sidestream fraction, separating the liquid sidestream fraction by decantation into an upper organic phase comprising principally entrainer and some methyl acetate and a lower aqueous phase comprising principally water and some methyl acetate and methanol, returning the separated organic phase to the column at a point lower in the column than the sidestream fraction removal point and passing the separated aqueous phase to a stripping column wherein a base aqueous fraction is separated from an overhead fraction, the base aqueous fraction being removed from the process and the overhead fraction being recycled to the esterification reactor and/or distillation column
characterised in that
the process is operated in 'standby' mode by the steps comprising:-
(i) shutting off the methanol and acetic acid feeds to the esterification reactor,
(ii) recycling substantially all the remaining methyl acetate separated overhead by distillation to the esterification reactor instead of passing it to downstream operation,
(iii)recycling the water in the lower aqueous phase from the decantation stage to the esterification reactor, and
(iv) adjusting the distillation column reflux to prevent dumping occurring on the distillation column.

6. A process as claimed in claim 5 in which in the 'standby' mode the lower aqueous phase is recycled directly to the esterification reactor, the stripping column either being shutdown or held on standby at or near reflux without feeds or take-offs.

7. A process as claimed in claim 5 in which in the 'standby' mode the lower aqueous phase is passed through the stripping column which continues to operate, the base water fraction being returned to the esterification reactor and the overhead fraction being returned to the esterification reactor and/or distillation column.

8. A process as claimed in any one of claims 5 to 7 in which the process is converted from 'standby' mode to operational mode by:
(a) restarting the methanol and acetic acid feeds to the esterification reactor;
(b) passing all the methyl acetate separated overhead to downstream operation;
(c) ceasing recycle of the water in the lower aqueous phase from the decantation stage to the esterification reactor and optionally restarting the stripping column if it has been placed on 'standby' or shutdown; and
(d) adjusting the distillation column reflux to its normal value.

9. A process for the production of acetic anhydride with or without the net co-production of acetic acid by carbonylation of methyl acetate charterised in that the methyl acetate is produced in a step integrated with the carbonylation reaction and in that the methyl acetate is produced in a step comprising continuously feeding methanol and acetic acid to an esterification reactor to form, at elevated temperature and in the presence of an esterification catalyst, a product comprising methyl acetate and water; recovering methyl acetate from the esterification product by distillation and passing the recovered methyl acetate, with or without further purification, to the carbonylation reaction and recovering water from the esterification product by azeotrope distillation and removing from the process the recovered water, with or without further purification; and further in that the methyl acetate production step is operated in 'standby' mode by the steps comprising:
(a) shutting off the methanol and acetic acid feeds to the esterification reactor;
(b) recycling the recovered methyl acetate to the esterification reactor instead of passing it to the carbonylation reaction; and
(c) recycling the recovered water to the esterification reactor instead of removing it from the process.
